(19) **Europäisches Patentamt**

**European Patent Office**

**Office européen des brevets**

(11) **EP 1 105 038 B1**

(12) **EUROPÄISCHE PATENTSCHRIFT**

(45) Veröffentlichungstag und Bekanntmachung des Hinweises auf die Patenterteilung:
**19.06.2002 Patentblatt 2002/25**

(21) Anmeldenummer: **99953576.8**

(22) Anmeldetag: **19.08.1999**

(51) Int Cl.⁷: $A61B\ 3/107$

(86) Internationale Anmeldenummer:
**PCT/DE99/02642**

(87) Internationale Veröffentlichungsnummer:
**WO 00/10449 (02.03.2000 Gazette 2000/09)**

(54) **VORRICHTUNG ZUR ERMITTLUNG DER OBERFLÄCHENFORM VON BIOLOGISCHEM GEWEBE**

DEVICE FOR DETERMINING THE SURFACE SHAPE OF BIOLOGICAL TISSUE

DISPOSITIF POUR DETERMINER LA FORME SUPERFICIELLE D'UN TISSU BIOLOGIQUE

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **20.08.1998 DE 19837932**

(43) Veröffentlichungstag der Anmeldung:
**13.06.2001 Patentblatt 2001/24**

(73) Patentinhaber: **Bioshape AG**
**12159 Berlin (DE)**

(72) Erfinder: **SCHRÜNDER, Stephan**
**D-12163 Berlin (DE)**

(74) Vertreter: **Schlief, Thomas P.**
**Friedrich-Ebert-Strasse 84**
**85055 Ingolstadt (DE)**

(56) Entgegenhaltungen:
**EP-A- 0 167 877          US-A- 4 995 716**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Verfahren und eine Vorrichtung zur Ermittlung der Oberflächenform von biologischem Gewebe gemäß den Oberbegriffen der Ansprüche 1 und 29.

Die exakte Kenntnis der Topologie von biologischen Geweben ist beispielswelse zur Durchführung von Operationen an der Gewebeoberfläche in vielen Fällen unerläßlich. Als Beispiel sei die Hornhautoberfläche des menschlichen Auges angeführt. Da die Hornhaut eine Brechkraft von über 40 Dioptrien besitzt, ist sie maßgeblich an der Brechung des in das Auge einfallenden Lichtes und somit am Sehvorgang beteiligt. Die Brechkraft hängt hierbei vorrangig von der Form der Hornhautoberfläche und insbesondere ihrer Kurvatur ab. Moderne Verfahren zur Korrektur von Fehlsichtigkeiten zielen daher auf eine Änderung der Hornhautform durch den Abtrag von Hornhautgewebe mit Hilfe eines Lasers ab. Voraussetzung für eine gezielte Bearbeitung der Hornhaut ist deshalb die genaue Kenntnis der Form ihrer äußeren Oberfläche. Diese wird derzeit vor und mehrere Tage nach der Fehlsichtigkeitskorrektur mit Hilfe optischer Verfahren ermittelt, bei denen die Meßwerte - aufgrund der Schnelligkeit dieser Verfahren - durch die statistischen und unwillkürlichen Bewegungen des Auges nicht nennenswert beeinflußt werden.

Ein bekanntes Verfahren zur Vermessung der Hornhautform, welches vor bzw. nach einer Fehlsichtigkeitsoperation oder auch zur Anpassung von Kontaktlinsen zum Einsatz kommt, stützt sich auf die Verwendung sog. Keratometer, bei denen die Reflexion von konzentrischen Ringen (sog. Placido-Ringe) an dem die Hornhaut benetzenden Tränenfilm mit einer Kamera aufgenommen und ausgewertet wird. Hierbei wird vor dem Auge eine Beleuchtungseinrichtung plaziert, vor welcher eine Scheibe mit kreisförmigen, zueinander konzentrischen Schlitzen angeordnet ist, in deren Zentrum wiederum eine Kamera aufgestellt wird. Das vom Tränenfilm reflektierte und von der Kamera aufgenommene Licht - in Form eines durch die Krümmung der Hornhaut verzerrten Ringmusters - wird zur Ermittlung der spezifischen Eigenarten der zu vermessenden Hornhautform mit einer vorgegebenen Hornhautform eines Standardauges, das einen Hornhautradius von 7,8 mm aufweist, verglichen. Zur Rekonstruktion der Oberflächenform der jeweiligen Hornhaut legt der Anwender zunächst manuell das Zentrum der zumeist ca. 20 Ringe mit Hilfe eines Fadenkreuzes fest. Danach werden durch das Zentrum 180 Meridiane über die Hornhaut mit einem Abstand von je 1° gelegt. Der Abstand der Schnittpunkte der Meridiane mit den Ringen nimmt mit wachsendem Radius der Ringe bis auf Werte um 300 μm zu. Insgesamt ergeben sich damit 180 (Meridiane) x 20 (Ringe) x 2 (Schnittpunkte) = 7200 Datenpunkte, aus denen die Krümmung der Hornhaut berechnet werden kann. Nachteilig bei diesem bekannten Verfahren bzw. dieser bekannten Vorrichtung ist, daß aufgrund der konzentrischen Anordnung der Beleuchtungseinrichtung und der Kamera keine Daten in einer Fläche des Zentrums mit einem Durchmesser von mindestens 1,5 mm aufgenommen werden können. Jedoch sind gerade in diesem Bereich Messungen besonders wichtig. Weiterhin lassen sich Fehlmessungen bei einer Hornhautform nicht vermeiden, die von der Form des Standardauges - wie beispielsweise bei einer zentralen Abflachung - stärker als üblich abweicht. Zudem ist die Anzahl von 7200 Datenpunkten für die zur Ermittlung der Hornhauttopologie notwendige Interpolation in manchen Fällen nicht befriedigend. Diese Anzahl an Datenpunkten stellt faktisch ein oberes Limit dar, da sich die Meridiane aufgrund ihrer endlichen Linienbreite nicht in geringerem Winkelabstand als 1° einteilen lassen.

Da das zuvor beschriebene Verfahren bzw. die entsprechende Vorrichtung keine Kontrolle während des Abtragvorgangs erlaubt, sind insbesondere bei hohen Fehlsichtigkeiten über -6 Dioptrien relativ häufig Fehlkorrekturen zu verzeichnen.

Zwar können diese Fehlkorrekturen vom Anwender bzw. Operateur statistisch zur Anfertigung sog. "Nomogramme" ausgewertet werden, die bei darauffolgenden operativen Eingriffen die Fehlkorrekturen im Mittel verhindern helfen; befriedigen kann diese Lösung jedoch nicht.

Des weiteren ist das industriell etablierte sog. Streifenprojektionsverfahren zur optischen Vermessung von Oberflächen verschiedenster lebloser Materialien bekannt, welches eine zuverlässige, berührungslose und schnelle Meßwerterfassung gestattet. Die grundlegende Idee der Streifenprojektionstechnik besteht in der Verbindung der meßtechnischen Möglichkeiten der geometrisch-optischen Triangulation mit denen der klassischen Interferometrie. Die mathematischen Zusammenhänge sind im Anhang näher dargestellt. Dieses Verfahren bzw. die entsprechende Vorrichtung sind besonders geeignet, schnelle Vorgänge zu erfassen, da nur eine einzige Aufnahme notwendig ist. Hierbei wird zunächst ein geeignetes Streifenmuster auf die zu vermessende Oberfläche projiziert. Die Streifen werden interferometrisch oder durch die Abbildung einer geeigneten Struktur (Gitter, Ätzstruktur in Glas, LCD-Matrix, Mikrospiegel) erzeugt. Das von der Oberfläche diffus gestreute Licht in Form eines durch die Oberflächenform der Hornhaut verzerrten Streifenmusters wird in einem Winkel $\alpha$ zur Projektions- bzw. Bestrahlungsrichtung detektiert und mittels geeigneter Algorithmen ausgewertet. Die erforderlichen und früher zeitintensiven Fouriertransformationen stellen aufgrund neuer Rechnermöglichkeiten keine nennenswerte Verzögerung mehr dar.

Problematisch wird die Auswertung der Streifenmuster jedoch bei relativ schwachem Kontrast des detektierten Streifenmusters. Dabei treten zuweilen Phasenmeßfehler auf, die sich in Sprüngen in der Fläche bemerkbar machen. Kontrasterhöhende Maßnahmen bestehen bekanntermaßen im Bedampfen des Objekts mit stark streuenden Schichten oder in der Zugabe von Fluoreszenzfarbstoffen. Letzteres ist insbesondere in der Augenheilkunde vorgeschlagen worden, so beispielsweise von Windecker et al. (Applied Optics 34, 3644 ff., 1995), die in einem Streifenprojektionsverfahren die Anreicherung des Tränenfilms vor der Hornhaut mit Fluorescin vorschlugen, um die Form der Hornhautoberfläche zu ermitteln. Bei diesem Verfahren wird blaues Licht, welches mittels eines Filters aus weißem Licht herausgefiltert wurde, auf die Hornhaut gelenkt, woraufhin der vorgelagerte, Fluorescin-angereicherte Tränenfilm als Folge der Anregung grünes Licht emittiert. In der US 54 06 342 ist ein ähnliches Verfahren (und eine entsprechende Vorrichtung) beschrieben, bei dem die Überlagerung von zwei Teilmustern, die auf die mit fluoreszierender Flüssigkeit versehene Hornhaut aus zwei Richtungen projiziert werden, zur Entstehung eines auswertbaren Moiré-Musters führt. Die vom Flüssigkeitsfilm abgestrahlte Fluoreszenzstrahlung wird nach Durchlaufen eines optischen Filters durch die sukzessive Aufnahme von zwei Halbbildern mit einer Videokamera vereinigt und durch speziell entwickelte Algorithmen ausgewertet. Die Projektion der Strahlung aus zwei Richtungen hilft - zusätzlich zu dem Filter -, die Detektion des direkten Reflexes zu vermeiden, welcher an dem Ort auf der Hornhaut entsteht, dessen Flächennormale den Winkel zwischen Bestrahlungs- und Beobachtungsrichtung in zwei gleich große Winkel teilt und immer dann in Erscheinung tritt, wenn die Detektionseinheit empfindlich für die das Muster projizierende Wellenlänge ist.

Weitere Verfahren bzw. Vorrichtungen zur Bestimmung der Hornhauttopographie, bei denen ein fluoreszierendes Mittel auf das Auge aufgebracht wird, sind aus der US-A-4,995,716, US-A-4,761,071 sowie US-A-5,159,361 bekannt.

Nachteilig bei diesen bekannten Verfahren bzw. Vorrichtungen ist, daß der Tränenfilm stets lokal und individuell unterschiedliche Dicken aufweist, so daß durch seine Vermessung nicht zuverlässig auf die Oberfläche der Hornhaut rückgeschlossen werden kann. Da sich das fluoreszierende Medium weiterhin im Tränenfilm verteilt und somit Streulicht aus der gesamten Dicke des Tränenfilms liefert, kann die Meßgenauigkeit nicht höher als die Filmdicke sein, die bis zu 200 μm beträgt. Weiterhin würde die Flüssigkeit bei nicht vorhandener oder aus dem Strahlengang weggeklappter Epithelschicht auf der Hornhaut in das Hornhautgewebe eindringen, woraus eine Verbreiterung der Tiefenauflösung resultieren würde. Außerdem würde sich in einem solchen Fall die Oberflächenform der Hornhaut verändern, da diese aufquillt. Somit ist für die Anwendung dieses bekannten Verfahrens bzw. dieser bekannten Vorrichtung eine intakte Epithelschicht erforderlich, die vor einer Operation jedoch gerade entfernt werden muß, so daß Messungen während einer Operation beispielsweise nicht möglich sind.

Des weiteren ist aus der GB-A-2 203 831 eine Vorrichtung zur Untersuchung von Tumoren mittels Fluoreszenstrahlung bekannt. Hierzu wird das zu untersuchende Gewebe (Tumor) mittels einer Lichtquelle, die Licht im UV-Bereich abstrahlt, bestrahlt. Das Gewebe wird hierdurch zur Emission von Fluoreszensstrahlung angeregt, die mittels eines Detektionssystems detektiert und anschließend gemessen wird. Die bekannte Vorrichtung eignet sich aber nur zur Untersuchung der Gewebeeigenschaften von biologischem Gewebe wie Tumoren, nicht jedoch zur Bestimmung der Oberflächenform des biologischen Gewebes.

Es ist Aufgabe der vorliegenden Erfindung, ein Verfahren bzw. eine Vorrichtung der eingangs genannten Art derart weiterzubilden, daß sich die Topologie eines biologischen Gewebes auf einfache Weise und in Abwesenheit jedweder, insbesondere fluoreszierender Flüssigkeiten ermitteln läßt und die Ergebnisse ggf. zur operativen Behandlung verwendet werden können.

Diese Aufgabe wird bei dem Verfahren der eingangs genannten Art dadurch gelöst, daß das Gewebe direkt mit einem mit Hilfe einer Anregungsstrahlung erzeugten Bestrahlungsmuster bestrahlt wird, so daß die bestrahlten Gewebebereiche zur Emission eines aus Fluoreszenzstrahlung bestehenden Fluoreszenzmusters angeregt werden, welches detektiert und zur Berechnung der Oberflächenform des Gewebes ausgewertet wird.

Weiterhin wird die Aufgabe bei einer Vorrichtung gemäß dem Oberbegriff des Anspruchs 29 dadurch gelöst, daß die Strahlungsquelle eine Anregungsstrahlung mit im wesentlichen im ultravioletten (UV) Wellenlängenbereich liegender Wellenlänge erzeugt.

Die Vorteile der Erfindung sind insbesondere darin zu sehen, daß kein dem biologischen Gewebe vorgelagerter und ggf. mit einer geeigneten Substanz angereicherter Film zur Emission von fluoreszierender Stahlung angeregt wird, sondern das biologische Gewebe selbst. Hierzu werden die Intensität und insbesondere die Wellenlänge der Anregungsstrahlung derart gewählt, daß ihre Eindringtiefe in das Gewebe gering ist und tatsächlich nur die äußersten Gewebebereiche (beispielsweise 2-3 μm) zur Fluoreszenz angeregt werden. Das zu delektierende Fluoreszenzmuster entspricht hierbei im wesentlichen dem zuvor auf das Gewebe projizierten Bestrahlungsmuster, verzerrt durch die ggf. gekrümmte Oberfläche des zu vermessenden Gewebes sowie dem Winkel zwischen der Beobachtungsrichtung und der Bestrahlungsrichtung. Die nicht bestrahlten Gewebebereiche werden hierbei nicht zur Emission von Fluoreszenzstrahlung angeregt. Da eine unerwünschte räumliche Verteilung der fluoreszierenden Materie - wie beispielsweise bei einem fluoreszierenden Flüssigkeitsfilm auf dem Gewebe - unterbleibt, können hierdurch keine Meßverfälschungen auftreten. Ebenso wird ein Aufquellen des Gewebes durch einen solchen vorgelagerten Flüssigkeitsfilm vermieden.

Insbesondere im Falle der Hornhaut kann bei dem erfindungsgemäßen Verfahren bzw. der erfindungsgemäßen Vorrichtung auf fluoreszierende oder andersartig markierende Flüssigkeiten zur Aufbringung auf das Auge verzichtet werden. Somit ist es in überraschend einfacher Weise möglich, die Hornhautoberfläche durch Fluoreszenzstrahlung direkt darzustellen, ohne den unpräzisen Umweg über einen vorgelagerten, fluoreszierenden Film einschlagen zu müssen.

Die Strahlungsquelle erzeugt erfindungsgemäß eine Anregungsstrahlung mit im wesentlichen im ultravioletten Wellenlängenbereich liegender Wellenlänge, In diesem Fall dringt die UV-Strahlung nur wenige Mikrometer in die Hornhaut ein (oberhalb des UV-Wellenlängenbereichs bis ins nahe Infrarot (IR) ist die Hornhaut transparent). Folglich stammt die von der Hornhaut emittierte Fluoreszenzstrahlung im wesentlichen aus der äußersten Gewebeschicht und repräsentiert somit deren Topologie hinreichend exakt. Weiterhin kann die Messung in ausreichend kurzer Zeit erfolgen, um Fehlmessungen aufgrund von Augenbewegungen zu vermeiden.

Mittels des erfindungsgemäßen Verfahrens bzw. der erfindungsgemäßen Vorrichtung lassen sich beispielsweise auch Deformationen an Extremitäten oder Oberflächenveränderungen und andere strukturelle Merkmale der Haut (beispielsweise Fingerabdrücke) vermessen. Gegebenenfalls ist es vorher angezeigt, im Lichtweg befindliche störende Objekte - wie beispielsweise Haare - von der Gewebeoberfläche zu entfernen. Die Form der Oberfläche des zu vermessenden Gewebes kann prinzipiell beliebig ausgebildet sein; allerdings sollten keine grobe Stufen vorhanden sein.

Die Auswerteeinheit zur Auswertung des Fluoreszenzmusters der Fluoreszenzstrahlung umfaßt bevorzugt einen Computer mit einer geeigneten Analysesoftware, die beispielsweise an sich bekannte mathematischen Methoden verwendet. Eine solche - an sich bekannte - mathematische Methode zur Auswertung der Fluoreszenzstrahlung ist im Anhang wiedergegeben.

Da das erfindungsgemäße Verfahren bzw. die erfindungsgemäße Vorrichtung auf den Fluoreszenzeigenschaften des Gewebes selbst basiert, ist es insbesondere auch geeignet, die Topologie während der refraktiven Operation an der Hornhaut zu erfassen, während der kein Tränenfilm und keine Epithelschicht - zumindest im Strahlengang der Anregungsstrahlung -vorhanden ist. Beispielsweise wird vor und während der Operation hinreichend oft die momentane Gewebetopologie bestimmt, so daß der nächste Operationsschritt auf das aktuelle Ergebnis abgestimmt werden kann. Dies gestattet somit die Steuerung/Regelung des Abtragprozesses während der Operation mit beispielsweise einem Laser, indem zwischen dem Operationsmodus und dem Modus zur Ermittlung der Oberflächenform der Hornhaut hin- und hergeschaltet wird, so daß eine exaktere Korrektur aufgrund ständigen Kontrollierens und entsprechenden Reagierens als bisher möglich ist. Dadurch werden auch individuell vom Anwender aus statistischen Untersuchungen festgelegte Nomogramme überflüssig, die insbesondere bei großen Korrekturen über -6 Dioptrien bislang noch erforderlich sind.

Besonders bevorzugt sind die Strahlungsquelle zur Bestimmung der Oberflächenform des biologischen Gewebes und diejenige zur operativen Behandlung des Gewebes identisch. Auf diese Weise läßt sich eine kompakte und relativ kostengünstige Vorrichtung zur Bestimmung der Gewebetopologie und Operation des Gewebes realisieren. Mit Hilfe des erfindungsgemäßen Verfahrens bzw. der erfindungsgemäßen Vorrichtung lassen sich somit schnell und einfach sehr präzise Gewebekorrekturen vornehmen. Die Aufgabe wird daher bei einem Verfahren zur Unterstützung eines operativen Eingriffs an einem biologischen Gewebe auch dadurch gelöst, daß das Ergebnis der Auswertung in die aktuelle operative Behandlung des biologischen Gewebes regelnd und/oder steuernd einbezogen wird.

Weiterhin ist vorteilhaft, daß die Detektion der Fluoreszenzstrahlung mit einer Vorrichtung erfolgen kann, deren Empfindlichkeit für die Anregungswellenlänge aufgrund des Wellenlängenunterschiedes zwischen der Anregungsstrahlung und der Fluoreszenzstrahlung a priori sehr gering ist. Hierbei bietet sich eine CCD-Kamera an, die eine ortsaufgelöste Detektion erlaubt. Es kann gegebenenfalls auch eine im Ultravioletten empfindliche Kamera verwendet werden. Zusätzlich oder alternativ läßt sich die Empfindlichkeit der jeweiligen Detektionsvorrichtung für die Anregungswellenlänge durch ein Filter (Farbfilter oder Polarisationsfilter) verringern. Auf diese Weise tritt der direkte Reflex bei der Detektion nicht störend in Erscheinung. Zwar läßt er sich ebenso wie bei den bekannten Verfahren bzw. Vorrichtungen nicht vermeiden; er wird aufgrund der in einem anderen Wellenlängenbereich liegenden Empfindlichkeit der Detektionsvorrichtung jedoch nicht detektiert und kann daher das gewünschte Signal nicht überdecken. Somit ist nur eine einzige Aufnahme zur vollständigen Rekonstruktion der Oberfläche des biologischen Gewebes erforderlich.

Vorzugsweise wird die vom biologischen Gewebe emittierte Fluoreszenzstrahlung unter einem von der Bestrahlungsrichtung verschiedenen Winkel detektiert, der beispielsweise 45° beträgt. Auf diese Weise wird das Fluoreszenzmuster perspektivisch verzerrt beobachtet, so daß insbesondere eine Krümmung der Gewebeoberfläche präziser vermessen werden kann. Die benachbarten Streifen eines Fluoreszenzstreifenmusters beispielsweise erscheinen bei einer derartigen Beobachtung aufgrund der Perspektive gekrümmter als bei einer Frontalbeobachtung, weshalb genauere Informationen über den Krümmungsverlauf erhalten werden können.

Falls die direkte Fluoreszenzstrahlung lediglich mit einer einzigen Detektionsvorrichtung aufgefangen wird und die Bestrahlungs- und die Beobachtungsrichtungen nicht zusammenfallen, erhält man - wie erörtert - ein perspektivisch verzerrtes Abbild des Fluoreszenzmusters. Bei einem sehr feinen Bestrahlungs- und damit Fluoreszenzmuster, das eine sehr genaue Auflösung der Gewebetopologie ermöglicht, können daher Linien, die in von der Detektionsvorrichtung abgewandten Bereichen liegen, unerwünschtermaßen zusammenfließen und sind dann nicht mehr präzise aufzulösen. Daher kann vorteilhafterweise mindestens eine weitere Detektionsvorrichtung verwendet werden, welche - bezüglich der Bestrahlungsrichtung - der ersten Detektionsvorrichtung beispielsweise gegenüberliegt und dazu dient, die Fluoreszenzstrahlung aus einem von der ersten Detektionsvorrichtung nicht präzise erfaßbaren Bereich des biologischen Gewebes zu detektieren. Alternativ kann auch ein geeignet vor dem biologischen Gewebe positionierter Spiegel verwendet werden, der die Fluoreszenzstrahlung von der der (einzigen) Detektionsvorrichtung abgewandten Seite des biologischen Gewebes zu dieser Detektionsvorrichtung spiegelt. Somit lassen sich beispielsweise zwei räumliche Halbbilder gleichzeitig (mit zwei Detektionsvorrichtungen) bzw. nacheinander (mit einer Detektionsvorrichtung und einem Spiegel) aufnehmen und zur Auswertung entsprechend vereinigen.

Zusätzlich oder alternativ wird das biologische Gewebe aus mindestens zwei Richtungen bestrahlt, um aufgrund der perspektivischen Verzerrung ansonsten schwer auszuleuchtende Gewebebereiche hinreichend zu erfassen. Vorteilhafterweise ist hierbei im Falle einer Hornhautvermessung ein symmetrischer Aufbau der erfindungsgemäßen Vorrichtung vorgesehen, wobei beispielsweise die beiden Projektions- bzw. Bestrahlungsrichtungen den gleichen Winkel mit einer zwischen ihnen verlaufenden Beobachtungsrichtung einschließen. Auch kann neben dem Einsatz von mehreren Strahlungsquellen die Verwendung von mehreren Detektionsvorrichtungen vorgesehen sein. Um das biologische Objekt von mehreren Seiten zu beleuchten bzw. zu bestrahlen, bieten sich ebenfalls Spiegel oder andere Lichtumlenkeinrichtungen an.

Das biologische Gewebe und insbesondere die Hornhaut wird vorzugsweise mit Wellenlängen zwischen 150 nm und 370 nm zur Fluoreszenz angeregt. Kürzere Wellenlängen als ca. 150 nm lassen sich derzeit nur mit hohem technischen Aufwand mit hinreichender Energie erzeugen. Außerdem erzeugen sie eine Fluoreszenzstrahlung, die sich mit konventioneller Technik aufgrund ihrer ebenfalls nur geringfügig längeren Wellenlänge schlecht detektieren ließe. Längere Wellenlängen als ca. 370 nm hingegen weisen - zumindest im Falle der Hornhaut für sichtbares Licht - eine zu große Eindringtiefe auf, um die Emission von Fluoreszenzstrahlung auf die äußersten Zellschichten zu beschränken und somit die erforderliche Genauigkeit der Messung zu gewährleisten. Außerdem könnten in diesem speziellen Fall Schädigungen am Auge auftreten.

Da im Falle der Hornhautvermessung das Auge unwillkürliche Bewegungen, sogenannte Sakkaden, vollzieht, ist es vorteilhaft, die Bestrahlungsdauer entsprechend zu beschränken, vorzugsweise unter 20 ms, da ansonsten das detektierte Fluoreszenzmuster verzerrt werden könnte. Neue Lasergeräte sind in der Lage, Pulse in der Größenordnung von Femtosekunden zu emittieren, die ggf. ebenfalls verwendet werden können.

Das genannte Problem der zwangsläufigen Begrenztheit der Bestrahlungsdauer aufgrund der Augenbewegungen kann bei dem erfindungsgemäßen Vefahren bzw. der erfindungsgemäßen Vorrichtung durch Einsatz mindestens eines Eye-Trackers umgangen werden, mittels welchem längere Bestrahlungszeiten zu realisieren sind. Der Eye-Tracker zeichnet hierbei die Bewegungsabläufe des Auges - vorteilhafterweise während der Bestrahlung mittels der Anregungsstrahlung - als Funktion der Zeit auf, die bei der Auswertung des Fluoreszenzmusters miteinbezogen werden. Die Anregungsstrahlung wird mittels der so erhaltenen Informationen dem Auge nachgeführt, um längere Bestrahlungszeiten zu realisieren.

Ferner ist vorzugsweise vorgesehen, eine Bestimmung der Lage des Auges mit einem Eye-Tracker, der dem o.g. Eye-Trakker durchaus entsprechen kann, vor jeder Bestrahlung mit dem Bestrahlungsmuster und nach jeder Detektion des Fluoreszenzmusters durchzuführen. Zusätzlich oder alternativ hierzu ist vorgesehen, die Lage des Auges während der Bestrahlung mit dem Bestrahlungsmuster und während der Detektion des Fluoreszenzmusters mittels eines Eye-Trackers zu ermitteln. Bei einer Änderung der Lage des Auges während der Bestrahlung mit dem Bestrahlungsmuster oder während der Detektion des Fluoreszenzmusters wird die Bestrahlung bzw. die Detektion gestoppt und eine erneute Bestrahlung der Hornhaut des Auges mit anschließender Detektion des Fluoreszenzmusters durchgeführt.

Es hat sich als vorteilhaft herausgestellt, wenn das Bestrahlungsmuster in kurzen zeitlichen Abständen auf das Gewebe geworfen wird. Mit einer solchen Bestrahlungspulsserie wird eine hinreichende Fluoreszenzintensität bei gleichzeitiger Schonung des Gewebes vor lichtinduziertem Abtrag realisiert. Bei schnell schaltenden optischen Elementen können mehrere hundert Strahlungspulse innerhalb der vorgegebenen Blitzdauer appliziert werden. Vorzugsweise liegt die Wiederholrate für jede Messung, die sich aus einer Bestrahlung mit anschließender Detektion des Fluoreszenzmusters zusammensetzt, zwischen 1 Hz und 1 MHz.

Um Hornhautgewebe oder anderes biologisches Gewebe durch die Anregungsstrahlung nicht dauerhaft zu beschädigen, wird bevorzugt die Projektion des geometrischen Bestrahlungsmusters mit einer hinreichend niedrigen Fluence (Energie/Fläche) vorgenommen. Diese sollte bei einer kreisförmigen Fläche mit einem Durchmesser von 10 mm weniger als 10 mJ betragen. Gleichfalls werden somit phototoxische Wirkungen vermieden. Die Energie der Anregungsstrahlung liegt vorzugsweise zwischen 1 µJ und 1 J.

Die die Fluoreszenz anregende Strahlung wird beispielsweise von einem Excimer-Laser emittiert, der auch zur operativen Bearbeitung der Hornhaut verwendet wird. Beispielsweise kommt ein ArF-Laser mit einer Wellenlänge von 193 nm zum Einsatz. Alternative Lasergeräte, die die Emission von Bestrahlungspulsen erlauben, sind neben Excimer-Lasern (ArF mit einer Wellenlänge $\lambda$=193 nm, KrF mit $\lambda$=248 nm, XeCl mit $\lambda$=308 nm, XeF mit $\lambda$=351 nm) und Stickstofflasern ($\lambda$=337 nm) auch frequenzvervielfachte Festkörperlaser (Nd:YAG 5-fach mit $\lambda$=213 nm bzw. 4-fach mit $\lambda$=266 nm bzw. 3-fach mit $\lambda$=355 nm oder Alexandrit) oder durch derartige Festkörperlaser gepumpte Farbstofflaser.

Vorteilhaft ist die Wahl einer Wellenlänge, bei der die Intensität der Fluoreszenzstrahlung möglichst hoch ist, da dadurch die Anforderungen an die Detektionsvorrichtung sinken.

Alternativ zu einem Laser bietet sich die Verwendung von kostengünstigeren Blitzlampen an, die mit Xenon- oder Deuterium-haltigen Gasgemischen gefüllt sind. Diese werden vorzugsweise durch geeignete Filter auf die Emission von UV-Strahlung begrenzt. Da die Ausgangsleistungen dieser Blitzlampen im UV zumeist niedriger als die von Lasern sind, müssen ggf. höhere Anforderungen an die Detektionsvorrichtung gestellt werden.

Das auf das biologische Gewebe, wie beispielsweise die Hornhaut, projizierte geometrische Bestrahlungsmuster besteht vorzugsweise aus parallelen Streifen mit sinus-, $\cos^2$- oder rechteckförmigem Intensitätsverlauf. Durch geeignete Algorithmen läßt sich somit beispielsweise bei einer Streifenbreite und einem Streifenabstand von 100 µm die Oberfläche mit einer Auflösung von wenigen Mikrometern vermessen. Alternativ kann ein Raster, dessen Schnittpunkte zur Auswertung herangezogen werden, ein Lochmuster, ein Muster aus mehreren konzentrischen Kreisllinien mit radial vom Zentrum ausgehenden und mit gleichem Winkelabstand angeordneten Linien, ein aus zwei Linienmustern bestehendes Moiré-Muster oder ein sonstiges geometrisches Muster gewählt werden.

Die Mittel zum Erzeugen des geometrischen Bestrahlungsmusters umfassen vorzugsweise eine Maske mit beispielsweise parallelen Schlitzen oder regelmäßig angeordneten Löchern, welche durch Bestrahlung mit der Anregungsstrahlung auf das Gewebe abgebildet werden. Die Intensitätsverluste sind bei diesen Mustern relativ gering, was insbesondere bei Bestrahlungssystemen mit verhältnismäßig geringer Leistung von Vorteil ist.

Alternativ lassen sich durch geeignete Präparation bereichsweise strukturell veränderte Substrate (z.B. Glas) als Mittel zum Erzeugen des Bestrahlungsmusters verwenden, bei denen sich beispielsweise Bereiche starker Streuung oder Absorption mit unpräparierten Bereichen hoher Transmission abwechseln. Derartige Substrate gestatten die Erzeugung eines sinusförmigen Intensitätsverlaufs.

Das Bestrahlungsmuster kann vorteilhafterweise auch durch an sich bekannte diffraktive optische Elemente, wie beispielsweise Mikrolinsen, erzeugt werden. Die Mikrolinsen, deren Durchmesser beispielsweise 100 µm beträgt, sind hierbei z.B. in einer engen, regelmäßigen Anordnung auf einem transparenten Glassubstrat aufgebracht, welches in den Strahlengang der Anregungsstrahlung plaziert wird, die beispielsweise einen Strahldurchmesser von 8 mm aufweist. Sind die einzelnen Mikrolinsen als Zylinderlinsen ausgebildet, lassen sich auf diese Weise Streifenmuster erzeugen. Auch andere Linsenformen - beispielsweise halbkugelförmige - sind möglich und definieren ein anderes Bestrahlungs- und damit Fluoreszenzmuster. Die Mikrolinsen erlauben es, eine höhere Tiefenschärfe zu erreichen, die bei der gekrümmten Hornhaut von Vorteil ist. Außerdem wird die Energie der Anregungsstrahlung besser genutzt als bei Verwendung einer Maske, welche einen Teil dieser Strahlung nicht zum Auge gelangen läßt. Weiterhin kann ein genauerer sinusförmiger Intensitätsverlauf von beispielsweise sich abwechselnden hellen und dunklen Streifen als bei einer Maske erzeugt werden.

Alternativ läßt sich das Bestrahlungsmuster auf dem biologischen Gewebe auch durch Interferenz erzeugen, indem die gegebenenfalls aufgeweitete Strahlung von einer monochromatischen, kohärenten Strahlungsquelle (Laser) durch einen Strahlteiler geschickt wird und auf dem biologischen Gewebe rekombiniert bzw. vereinigt wird. Alternativ läßt sich ein Interferenzmuster auf dem Gewebe mittels zweier aufeinander abgestimmter Strahlungsquellen mit zueinander kohärenter Strahlung erzeugen.

Das Bestrahlungsmuster läßt sich auch durch ein Feld aus Mikrospiegeln erzeugen, an denen die Anregungsstrahlung in geeigneter Weise zum Gewebe reflektiert wird. Solche Spiegel zeichnen sich durch kurze Einstellzeiten und die Erzeugung individueller Bestrahlungsmuster aus. Beispielsweise sind 300 x 400 Mikrospiegel gleichmäßig nebeneinander angeordnet.

Das auf dem biologischen Gewebe erzeugte Bestrahlungsmuster kann sich auch aus mehreren Teilmustern zusammensetzen, die auf eine oder mehrere der oben genannten Arten erzeugt werden.

Die strukturierte Fluoreszenzstrahlung wird vorzugsweise durch eine hinreichend empfindliche CCD-Kamera mit hoher räumlicher Auflösung (hoher Pixelzahl, beispielsweise 1280 x 1024 Pixel) aufgenommen, die eine ortsaufgelöste Detektion des Fluoreszenzmusters ermöglicht (eine ortsaufgelöste Detektion ist aufgrund der relativen Einfachheit der Auswertung sowie ihrer Präzision von besonderem Vorteil). Auf diese Weise lassen sich einige Hunderttausend auswertbare Datenpunkte erhalten. Falls die Anregungsstrahlung zudem im UV-Bereich liegt und die Fluoreszenzstrahlung im Sichtbaren, bietet sich eine CCD-Kamera vorteilhafterweise auch deshalb an, weil ihre Empfindlichkeit im sichtbaren Wellenlängenbereich höher ist als im ultravioletten Bereich. Zudem wirkt das Kameraobjektiv der üblichen CCD-Kameras als Bandpaßfilter für Wellenlängen oberhalb von 300 nm, so daß Fluoreszenzstrahlung oberhalb dieser Wellenlänge detektiert wird, nicht hingegen die vom Gewebe reflektierte Anregungsstrahlung, falls diese im kürzerwelligen UV-Bereich liegt.

Die CCD-Kamera kann bei relativ schwachen Strahlungstintensitäten - wie auch bei der Verwendung von Blitzlampen - mit einem Verstärker verbunden werden.

Vorteilhafte Weiterbildungen der Erfindung sind durch die Merkmale der Unteransprüche gekennzeichnet.

Im folgenden wird ein Ausführungsbeispiel der Erfindung anhand der Zeichnungen näher erläutert. Es zeigen:

Fig. 1    einen schematischen Aufbau einer ersten Ausführungsform einer Vorrichtung zur Projektion eines Bestrahlungsmusters auf eine Hornhaut und zur Detektion des erzeugten Fluoreszenzmusters;

Fig. 2    einen schematischen Aufbau eines zweiten Ausführungsform zur Musterprojektion und -detektion;

Fig. 3    ein auf einer Augenhornhaut mit einer CCD-Kamera beobachtetes Fluoreszenzmuster, das durch Bestrahlung der Hornhaut mit einem entsprechenden Bestrahlungsmuster gemäß Fig. 1 erzeugt wurde; und

Fig. 4    eine vereinfachte Darstellung der Strahlverläufe (zur Erläuterung der im Anhang wiedergegebenen mathematischen Ableitungen)

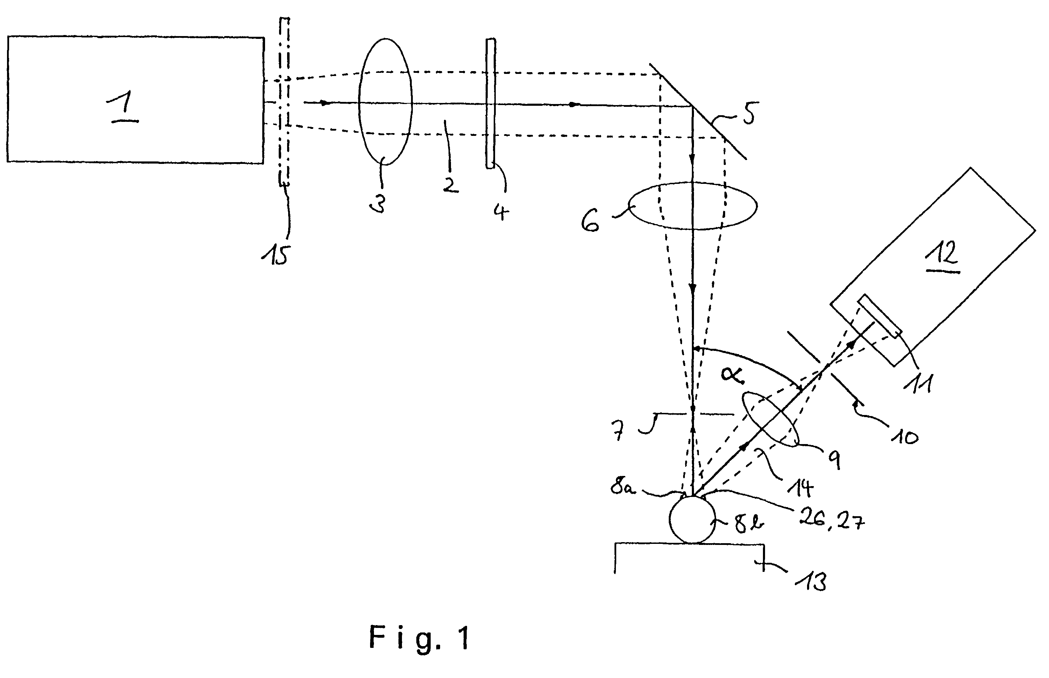

In Fig. 1 ist eine erste Ausführungsform der erfindungsgemäßen Vorrichtung dargestellt. Eine Strahlungsquelle 1 erzeugt eine Anregungsstrahlung 2, vorzugsweise eine UV-Strahlung. Da diese nicht notwendigerweise kollimiert ist, sorgt ein optionales erstes Linsensystem 3 (angedeutet durch eine schematisch dargestellte Sammellinse) für einen parallelen und homogenen Strahl. Dieser Strahl durchläuft Mittel 4 zum Erzeugen eines Bestrahlungsmusters 26, welche in der dargestellten Ausführungsform von einer senkrecht zum Strahlengang aufgestellten Schlitzblende bzw. Maske 4 mit beispielsweise parallelen streifenförmigen Öffnungen mit einer Breite und einem jeweiligen Abstand von 100 µm (nicht dargestellt) gebildet sind. Die Anregungsstrahlung 2, von der in Fig. 1 lediglich der Mittelpunktsstrahl als durchgezogene Linie mit Andeutung der Strahlungsrichtung sowie die Einhüllenden bzw. die Randstrahlen als gestrichelte Linien dargestellt sind, wird an dieser Maske 4 teilweise zurückgehalten und teilweise - durch die Öffnungen - durchgelassen. Auf diese Weise wird die Anregungsstrahlung 2 quer zur Strahlungsrichtung in Form eines Bestrahlungsmusters 26 strukturiert, welches im weiteren Strahlverlauf an einem Spiegel 5 umgelenkt und mittels eines zweiten Linsensystems 6 (angedeutet durch eine schematisch dargestellte Sammellinse) nach Durchlaufen einer ersten Aperturblende 7 auf die Oberfläche eines biologischen Gewebes 8a abgebildet wird. Das Gewebe 8a in dem gewählten Ausführungsbeispiel ist beispielsweise die Augenhornhaut 8a eines menschlichen Patienten, der auf der Patientenliege 13 plaziert ist (der Einfachheit halber ist nur das Auge 8b des Patienten dargestellt).

Die die Maske 4 passierende Anregungsstrahlung 2 ist hinsichtlich Intensität und Wellenlänge so gewählt, daß sie nur wenige Mikrometer in die Hornhaut 8a eindringt. Dies ist z.B der Fall, wenn ihre Wellenlänge im UV-Bereich liegt. Die Anregungsstrahlung 2 regt die Hornhaut 8a zur Emission von Fluoreszenzstrahlung 14 in den bestrahlten Bereichen an, während die nicht bestrahlten Bereiche der Hornhaut 8a keine Fluoreszenzstrahlung 14 emittieren können. Auf diese Weise emittiert die Hornhaut 8a ein dem Bestrahlungsmuster 26 entsprechendes, durch die Hornhautkrümmung verzerrtes Fluoreszenzmuster 27, welches unter einem Winkel $\alpha$ mit Hilfe eines dritten Linsensystems 9 nach Durchlaufen einer zweiten Aperturblende 10 auf den Sensor 11 einer Detektionsvorrichtung 12 abgebildet wird. Die Detektionsvorrichtung 12 ist beispielsweise eine ggf. durch einen Bildverstärker (nicht dargestellt) intensivierte CCD-Kamera 12. Es genügt eine Aufnahme mittels der Detektionsvorrichtung 12, um die gesamte benötigte Information über die Oberflächenform der Hornhaut zu erhalten. Hierzu ist die Detektionsvorrichtung 12 mit einer vorzugsweise von einem Computer gebildeten Auswerteeinheit (nicht dargestellt) verbunden, die mit Hilfe von Auswerteprogrammen die Form der Hornhaut 8a berechnet.

Die Wellenlänge der Anregungsstrahlung 2 liegt z.B. bei Verwendung eines ArF-Lasers als Strahlungsquelle 1 bei 193 nm. Ein ebenfalls bevorzugt eingesetzter frequenzverfünffachter Nd:YAG-Laser emittiert Anregungsstrahlung 2 der Wellenlänge 213 nm. Die Hauptmaxima der von den bestrahlten Gewebebereichen der Hornhaut 8a ausgehenden Fluoreszenzstrahlung 14 liegen in diesen Fällen ungefähr bei 300 nm und 450 nm, die einer Detektion ohne größeren Aufwand - wie beispielsweise mittels der CCD-Kamera 12 - zugänglich sind.

Auf der Hornhaut 8a sollte möglichst kein Tränenfilm vorhanden sein, um eine Absorption der Anregungsstrahlung 2 durch den Tränenfilm zu verhindern. Diese Notwendigkeit fügt sich sehr gut mit den erforderlichen Bedingungen während einer Operation an der Hornhaut 8a, die in der Regel am tränenfilmfreien Auge 8b durchgeführt wird. Das vorgestellte Verfahren bzw. die vorgestellte Vorrichtung eignet sich daher insbesondere zur Kombination einer wechselweisen Vermessung der Form der Hornhaut 8a sowie ihrer operativen Bearbeitung, welche vorteilhafterweise mit derselben Strahlungsquelle 1, üblicherweise einem UV-Laser, vorgenommen wird. Die Ergebnisse der Bestimmung der Hornhautform können dann sofort im anschließenden Operationsschritt verwendet werden, um die Hornhautabtragung mittels des Lasers zu steuern bzw. zu regeln. Während einer anschließenden Vermessungsphase kann sofort das Resultat des vorangehenden operativen Schritts kontrolliert und der nächste Operationsschritt darauf abgestimmt werden. Vorzugsweise wird dieser wechselseitige Vorgang automatisch mit Hilfe eines Computers gesteuert.

Bei der Verwendung eines Laserstrahls, der die Hornhaut 8a großflächig abzutragen vermag, wird während der Vermessung der Hornhautoberfläche zur Schonung der Hornhaut 8a mindestens ein Intensitätsabschwächer 15 (in Fig. 1 strichpunktiert dargestellt) in den Strahlengang der Anregungsstrahlung 2 - also zwischen der Strahlungsquelle 1 und der Hornhaut 8a - eingefügt, der während der Operationsphasen wieder aus dem Strahlengang entfernt wird. Das Einfügen und das Entfernen des Intensitätsabschwächers 15 in den Strahlengang wird vorzugsweise computergesteuert durchgeführt.

Bei einer weiteren, nicht dargestellten Ausführungsform wird ein Laserstrahl mit einem Durchmesser von beispielsweise 2 mm verwendet, um die Hornhaut 8a in jeweils nur kleinen Bereichen abzutragen. Hierbei wird der Laserstrahl scannend über die Hornhaut 8a geführt. Während der Vermessung der Hornhautoberfläche muß daher der Laserstrahl zur Erzeugung des großflächigeren Bestrahlungsmusters 26 mit mindestens einem Strahlaufweiter (nicht dargestellt) aufgeweitet werden, welcher zwischen die Strahlungsquelle 1 und die Hornhaut 8a in den Strahlengang eingebracht wird. Während der Operationsphasen wird der mindestens eine Strahlaufweiter aus dem Strahlengang der Anregungsstrahlung 2 wieder entfernt.

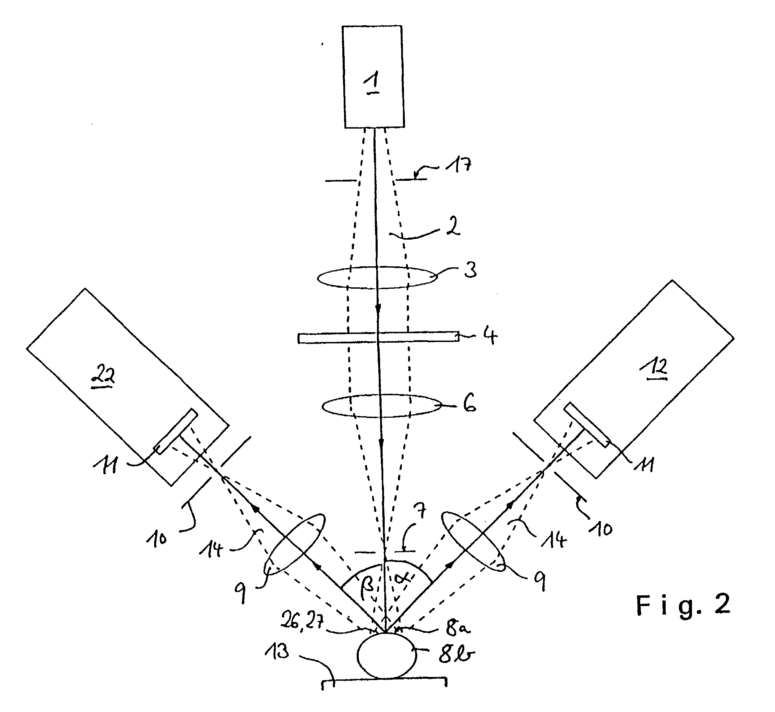

In dem in Fig. 2 dargestellten zweiten Ausführungsbeispiel der Vorrichtung sind zwei Detektionsvorrichtungen 12, 22 sich gegenüberliegend vor der Hornhaut 8a angeordnet, wobei die Beobachtungsrichtungen - verdeutlicht durch die jeweiligen zentralen Verbindungslinien zwischen der Hornhaut 8a und den Detektionsvorrichtungen 12, 22 - im dargestellten Fall die gleich großen Winkel $\alpha$, $\beta$ mit der Bestrahlungsrichtung einschließen, die mit dem Mittelpunktsstrahl der Anregungsstrahlung 2 zusammenfällt. Bei diesem Ausführungsbeispiel liegt die Strahlungsquelle 1 der Hornhaut 8a direkt gegenüber. Ähnlich dem ersten Ausführungsbeispiel gemäß der Fig. 1 fällt das Bestrahlungsmuster 26 nach Passieren einer dritten Aperturblende 17, des ersten Linsensystems 3, der Mittel 4 zum Erzeugen des Bestrahlungsmusters 26 sowie des zweiten Linsensystems 6 auf die Hornhaut 8a eines menschlichen Auges 8b. Die Fluoreszenzstrahlung 14 des Fluoreszenzmusters 27 wird in diesem Ausführungsbeispiel nach Durchlaufen von jeweils dritten Linsensystemen 9 und zweiten Aperturblenden 10 von zwei Seiten delektiert, um eine höhere Auflösung zu erhalten. Auf diese Weise ist - bei einer gekrümmten Gewebeoberfläche - auch die der jeweiligen Detektionsvorrichtung 12, 22 abgewandte Gewebeseite mit der jeweils anderen Detektionsvorrichtung 22, 12 präzise topographisch bestimmbar. Falls beispielsweise das Bestrahlungsmuster 26 von regelmäßig angeordneten, beabstandeten Kreisen gebildet ist, die auf den Kreuzungspunkten eines imaginären Quadratgitters liegen, rücken die Kreise aufgrund der perspektischen Verzerrung auf der der Detektionsvorrichtung 12 abgewandten Seite zusammen, bis sie sogar eventuell nicht mehr aufzulösen sind. Diesen Gewebebereich kann dann die ihm gegenüberliegende Detektionsvorrichtung 22 präzise erfassen. Entsprechendes gilt - mit vertauschten Rollen - für den der Detektionsvorrichtung 22 abgewandten Gewebebereich.

Bei einer weiteren, nicht dargestellten Ausführungsform wird das Gewebe 8a aus zwei Richtungen bestrahlt. Beispielsweise spaltet ein Strahlteiler die Anregungsstrahlung 2 von einer Strahlungsquelle 1 auf und lenkt sie mit Hilfe einer oder mehrerer Lichtumlenkeinrichtungen - wie beispielsweise Spiegel - auf das Gewebe 8a. Alternativ werden mehrere Stahlungsquellen 1 verwendet. Ein solcher Aufbau könnte z.B. wie derjenige in Fig. 2 aussehen, nur daß die beiden Detektionsvorrichtungen 12, 22 in Fig. 2 gegen zwei Strahlungsquellen 1 und die Strahlungsquelle 1 in Fig. 2 gegen eine Detektionsvorrichtung 12 auszutauschen wäre; selbstverständlich wären auch die Mittel 4 zum Erzeugen des Bestrahlungsmusters 26 sowie die Linsensysteme 3, 6, 9 und die Aperturblenden 7, 10, 17 entsprechend umzupositionieren.

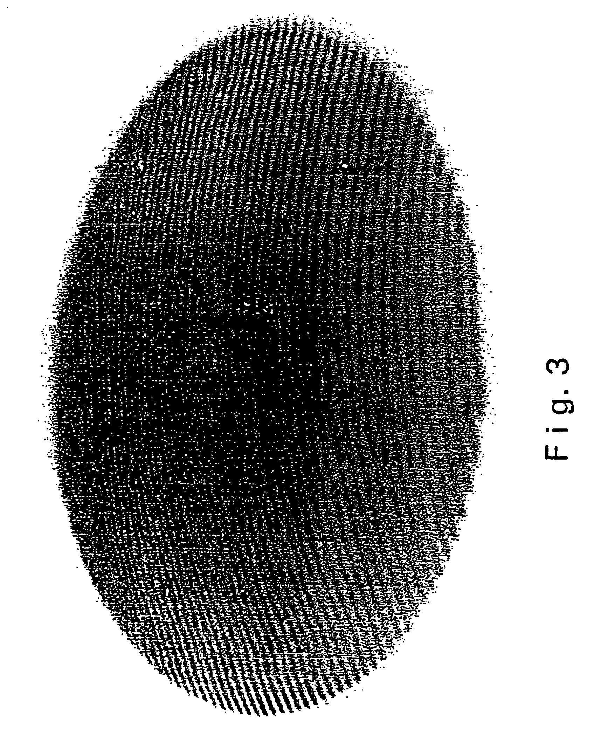

In Fig. 3 ist ein von einer CCD-Kamera 12 aufgenommenes, invertiertes streifenförmiges Fluoreszenzmuster 27 dargestellt, das während der Bestrahlung der Hornhaut 8a mit einem streifenförmigen Bestrahlungsmuster 26 gemäß dem Versuchsaufbau der Fig. 1 detektiert wurde. Das Bestrahlungsmuster 26 wurde hierbei mit Hilfe eines ArF-Excimerlasers ($\lambda$=193 nm) und einer Maske 4 mit parallelen Öffnungen erzeugt und nur auf denjenigen zentralen Bereich der Hornhaut 8a projiziert, der dem üblicherweise bei einer Laseroperation zu bearbeitenden, d.h. unterschiedlich stark abzutragenden Bereich entspricht. Die auf die Hornhaut 8a auftreffende Energie betrug hierbei ungefähr 2 mJ, während der Laserstrahl einen Durchmesser von 8 mm aufwies. Die sich unmittelbar an diesen Bereich anschließenden unbeleuchteten Nachbarbereiche 18 sind ebenfalls noch Teil der Hornhaut 8a. Gegebenenfalls kann eine digitale Subtraktion der vor und während der Einstrahlung des Bestrahlungsmusters 26 aufgenommenen Bilder den Kontrast und damit die Präzision des Verfahrens noch steigern.

Auf der Hornhaut 8a befand sich während der Aufnahme des in Fig. 3 gezeigten Fluoreszenzmusters 27 kein extern aufgebrachter Flüssigkeitsfilm bzw. Tränenfilm; die obersten Schichten der Hornhaut 8a wurden in den mit dem Bestrahlungsmuster 26 bestrahlten Bereichen direkt zur Emission von Fluoreszenzstrahlung 14 mit Hilfe der Anregungsstrahlung 2 angeregt. Zudem war vorher die Epithelschicht auf der Hornhaut 8a entfernt worden. Alternativ wird die Epithelschicht nach entsprechendem Einritzen mit einem Teil des darunter liegenden Stroma aus dem Strahlengang der Anregungsstrahlung weggeklappt und nach der Operation wieder in die ursprüngliche Position gebracht.

Die in der Fig. 3 nach oben enger zusammenrückenden Streifen sind eine Folge der perspektischen Verzerrung aufgrund der gegenüber der Bestrahlungsrichtung geneigten Beobachtungsrichtung bzgl. des Auges 8b (s. Fig. 1; dort entspricht die der Dektionsvorrichtung 12 zugewandte Gewebeseite dem unteren Bereich des Fluoreszenzmusters 27 in Fig. 3, während die der Detektionsvorrichtung 12 abgewandte Gewebeseite dem oberen Bereich des Fluoreszenzmusters 27 entspricht).

Während die oben aufgeführten Ausführungsbeispiele jeweils hinsichtlich der Vermessung der Oberflächenform einer Augenhornhaut erläutert wurden, sind das erfindungsgemäße Verfahren bzw. die erfindundungsgemäße Vorrichtung ohne Einschränkung ebenfalls geeignet, in entsprechender Weise an anderen biologischen Geweben eingesetzt zu werden.

Anhang

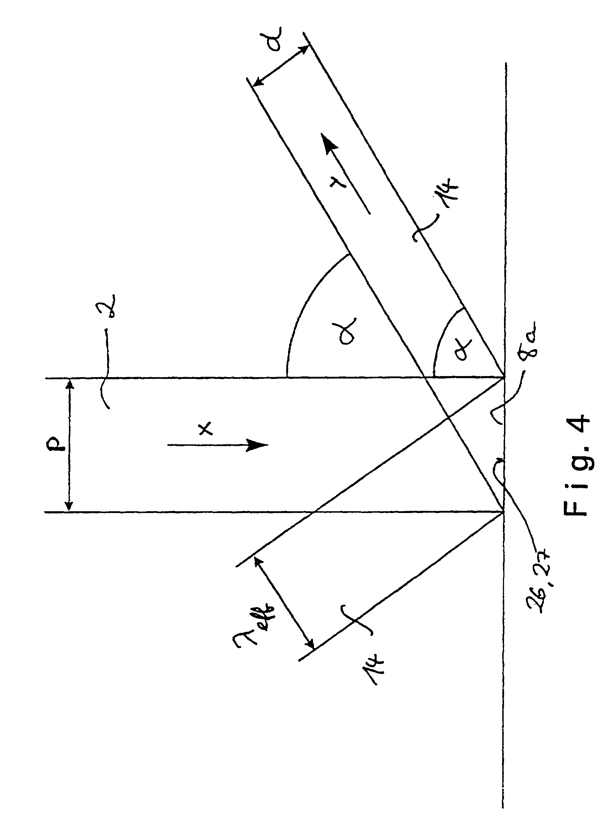

Im folgenden werden unter Bezugnahme auf die Fig. 4 die wesentlichen mathematischen Zusammenhänge erläutert, die für die Auswertung der emittierten Fluoreszenzstrahlung bei einem auf ein biologisches Gewebe 8a projizierten Streifenmuster (das Bestrahlungsmuster) ausgenutzt werden. Die Pfeile x bzw. y in Fig. 4 deuten jeweils die Bestrahlungsbzw. Beobachtungsrichtung an. Mit p ist der auf dem Objekt bzw. dem Gewebe 8a erzeugte Streifenabstand bezeichnet. Die Größe d ist der Abstand der Streifen, wie er vom Beobachter unter dem Winkel $\alpha$ wahrgenommen wird. Die effektive Wellenlänge, die eine der wesentlichen Größen bei der Auswertung des Streifenmusters darstellt, ist mit $\lambda_{eff}$ bezeichnet.

Eine wesentliche Größe ist der Abstand der Streifen senkrecht zur Beobachtungsrichtung, der auch als die effektive Wellenlänge $\lambda_{eff}$ bezeichnet wird und sich in Anlehnung an Fig. 4 nach der folgenden Formel berechnen läßt:

$$\lambda_{eff} = p \, / \sin \alpha = d \, / \tan \alpha = \beta \, L \, / \sin \alpha \qquad (1)$$

In Gleichung 1 ist $\alpha$ der Winkel zwischen Bestrahlungs- und Beobachtungsrichtung. L ist der Streifenabstand auf dem Bestrahlungsmuster, das über das Linsensystem 6 mit dem Vergrößerungsfaktor $\beta$ auf der Oberfläche des Objekts bzw. dem Gewebe 8a abgebildet wird. Die Größe d ist der Streifenabstand aus der Perspektive des Beobachters.

Die effektive Wellenlänge $\lambda_{eff}$ ist analog zur Interferometrie eine wichtige Größe, nach der sich die Empfindlichkeit des Systems bestimmt. Aus Gleichung 1 ist ersichtlich, daß sich $\lambda_{eff}$ durch die Variation des Winkels $\alpha$ und den Streifenabstand L verändern läßt. Je nach verwendetem Projektionsverfahren beträgt die Höhenauflösung bis zu $\lambda/100$.

Wird ein Projektionsgitter mit einem $\cos^2$-förmigen Intensitätsverlauf verwendet, so entsteht durch dessen Projektion auf der Oberfläche des Meßobjekts bzw. dem Gewebe 8a eine Intensitätsstruktur I(x,y), die durch Gleichung 2 beschrieben werden kann:

$$I(x,y) = I_0(x,y) + V(x,y) \cos \phi(x,y) \qquad (2)$$

Dabei ist $I_0(x,y)$ die Hintergrundintensität, V(x,y) der Streifenkontrast und $\phi(x,y)$ die Interferenzphasenlage. Der Phasenterm $\phi(x,y)$ stellt den Zusammenhang zwischen den parallelen Streifenlinien und dem Konturverlauf der Oberfläche des Objekts bzw. des Gewebes 8a dar. Der Konturverlauf ergibt sich quantitativ aus den Gleichungen 1 und 2 zu

$$z(x,y) = [\phi(x,y) \, \lambda_{eff}] \, / \, 4\pi \qquad (3)$$

Nach Gleichung 3 ist die schnelle und präzise Messung der Phase zur Bestimmung des Konturverlaufs unabdingbar. Aus der Echtzeitinterferometrie stehen hierfür eine Reihe sehr effektiver Phasenmeßverfahren zur Verfügung, die diese Aufgabe auf der Grundlage von Gleichung 2 erfüllen.

Als Phasenschiebeverfahren bezeichnete Auswertemethoden bauen darauf auf, daß im Streifenmuster durch die Veränderung von optischen Parametern über dem gesamten Bild eine schrittweise, definierte Veränderung der Phasenlage um genau eine Periode vorgenommen wird. Dies geschieht beispielsweise durch das Verschieben des Referenzspiegels eines Interferometers oder das Verschieben des Projektionsgitters. Dieses Meßverfahren liefert eine Genauigkeit von bis zu 1/100 der verwendeten Wellenlänge. Es zeichnet sich allerdings durch einen oft nur schwer zu realisierbaren technischen Aufwand und durch einen längeren Meß- und Auswertevorgang im Vergleich zur Einzelbildprojektion und -auswertung aus.

Daher ist es von Vorteil, wenn die Phasenermittlung nur eine Aufnahme erfordert. In der Echtzeitinterferometrie werden hierfür räumliche Heterodyn- oder Trägerfrequenzverfahren verwendet. Dabei wird dem Signal eine Ortsträgerfrequenz $f_0$ aufgeprägt, so daß sich Gleichung 2 ändert in:

$$I(x,y) = I_0(x,y) + V(x,y) \cos \{2\pi \, f_0 \, x + \phi(x,y)\} \qquad (4)$$

Mit der Gleichung

$$c(x,y) = \tfrac{1}{2}\, V(x,y)\, \exp\{i\,\phi(x,y)\} \qquad (5)$$

ergibt sich Gleichung 4 zu

$$I(x,y) = I_0(x,y) + c(x,y)\, \exp\{2\pi\, i\, f_0\, x\}$$

$$+\, c^*(x,y)\, \exp\{-2\,\pi\, i\, f_0\, x\} \qquad (6)$$

Dabei bedeutet * komplex konjugiert.

Die Funktion c(x,y) erhält man daraus über eine Fouriertransformation und eine Rücktransformation nach Filterung. Die Phase ergibt sich sodann zu

$$\phi(x,y) = \tan^{-1}\{\mathrm{Im}[c(x,y)]\,/\,\mathrm{Re}[c(x,y)]\} \qquad (7)$$

Hier sind "Im" der Imaginär- und "Re" der Realteil der komplexen Funktion c(x,y).

**Patentansprüche**

**1.** Verfahren zur Bestimmung der Oberflächenform von biologiechem Gewebe, **dadurch gekennzeichnet, daß** das Gewebe (8a) direkt mit einem mit Hilfe einer Anregungsstrahlung (2) erzeugten Bestrahlungsmuster (26) bestrahlt wird, so daß die bestrahlten Gewebebereiche zur Emission eines aus Fluoreszenzstrahlung (14) bestehenden Fluoreszenzmusters (27) angeregt werden, welches detektiert und zur Berechnung der Oberflächenform des Gewebes (8a) ausgewertet wird.

**2.** Verfahren nach Anspruch 1, **dadurch gekennzeichnet, daß** das zu vermessende Gewebe (8a) die Hornhaut (8a) eines Auges (8b) oder die Haut an einer Fingerkuppe ist.

**3.** Verfahren nach Anspruch 2, **dadurch gekennzeichnet, daß** vor der Bestimmung der Oberflächenform der Hornhaut (8a) der Tränenfilm auf der Hornhaut (8a) entfernt wird.

**4.** Verfahren nach Anspruch 3, **dadurch gekennzeichnet, daß** vor der Bestimmung der Oberflächenform der Hornhaut (8a) die Epithelschicht der Hornhaut (8a) zumindest vorübergehend derart aus dem Strahlengang der Anregungsstrahlung (2) entfernt wird, daß das unter der Epithelschicht liegende stromale Gewebe direkt mit dem Bestrahlungsmuster (26) bestrahlt wird.

**5.** Verfahren nach mindestens einem der vorangehenden Ansprüche, **dadurch gekennzeichnet, daß** die die Fluoreszenzstrahlung (14) emittierenden Gewebebereiche im wesentlichen mit einer im ultravioletten (UV) Wellenlängenbereich liegenden Anregungsstrahlung (2) angeregt werden.

**6.** Verfahren nach mindestens einem der vorangehenden Ansprüche, **dadurch gekennzeichnet, daß** das biologische Gewebe (8a) aus mindestens zwei Richtungen mit der Anregungsstrahlung (2) bestrahlt wird.

**7.** Verfahren nach mindestens einem der vorangehenden Ansprüche, **dadurch gekennzeichnet, daß** die Fluoreszenzstrahlung (14) mit mindestens einer Detektionsvorrichtung (12, 22), vorzugsweise einer CCD-Kamera (12, 22), detektiert wird.

**8.** Verfahren nach Anspruch 7, **dadurch gekennzeichnet, daß** die Fluoreszenzstrahlung (14) ortsaufgelöst delektiert wird.

**9.** Verfahren nach mindestens einem der vorangehenden Ansprüche, **dadurch gekennzeichnet, daß** die Fluoreszenzstrahlung (14) unter einem von der Bestrahlungsrichtung verschiedenen Winkel ($\alpha$) detektiert wird.

**10.** Verfahren nach mindestens einem der vorangehenden Ansprüche, **dadurch gekennzeichnet, daß** die Fluoreszenzstrahlung (14) zumindest tellweise von einer Lichtumlenkvorrichtung zu einer Detektionsvorrichtung (12, 22) umgeleitet und dort detektiert wird.

11. Verfahren nach mindestens einem der vorangehenden Ansprüche. **dadurch gekennzeichnet, daß** die Anregungsstrahlung (2) im Wellenlängenbereich von 150 nm bis 370 nm gewählt wird.

12. Verfahren nach mindestens einem der vorangehenden Ansprüche, **dadurch gekennzeichnet, daß** die Emissionsdauer der Anregungsstrahlung (2) zwischen 1 fs und mehreren Sekunden gewählt wird.

13. Verfahren nach mindestens einem der Ansprüche 2 bis 12, **dadurch gekennzeichnet, daß** ein Eye-Tracker informationen über typische Bewegungsabläufe des Auges aufzeichnet, mit deren Hilfe die Anregungsstrahlung (2) dem Auge (8b) nachgeführt wird, um lange Bestrahlungszeiten zu realisieren.

14. Verfahren nach mindestens einem der vorangehenden Ansprüche 2 bis 12, **dadurch gekennzeichnet, daß** eine Lagebestimmung des Auges (8b) mit einem Eye-Tracker vor jeder Betrahlung mit dem Bestrahlungsmuster (26) und nach jeder Detektion des Fluoreszenzmusters (27) durchgeführt wird.

15. Verfahren nach mindestens einem der Ansprüche 2 bis 12, **dadurch gekennzeichnet, daß** die Lage des Auges (8b) während der Bestrahlung mit dem Bestrahlungsmuster (26) und während der Detektion des Fluoreszenzmusters (27) mittels eines Eye-Trackers ermittelt wird und daß bei einer Änderung der Lage während der Bestrahlung mit dem Bestrahlungsmuster (26) oder während der Detektion des Fluoreszenzmusters (27) die Bestrahlung oder die Detektion gestoppt und eine emeute Bestrahlung der Hornhaut (8a) des Auges (8b) mit anschließender Detektion des Fluoreszenzmusters (27) durchgeführt wird.

16. Verfahren nach mindestens einem der vorangehenden Ansprüche, **dadurch gekennzeichnet, daß** das Bestrahlungsmuster (26) für jede Messung, die sich aus einer Bestrahlung mit anschließender Detektion des Fluoreszenzmusters (27) zusammensetzt, auf das Gewebe (8a) mit einer Wiederholrate zwischen 1 Hz und 1 MHz projiziert wird.

17. Verfahren nach mindestens einem der vorangehenden Ansprüche, **dadurch gekennzeichnet, daß** die Energie der Anregungsstrahlung (2) zwischen 1 μJ und 1 J gewählt wird.

18. Verfahren nach mindestens einem der vorangehenden Ansprüche, **dadurch gekennzeichnet, daß** die Anregungsstrahlung (2) mit einer als Laser (1) oder Blitzlampe ausgebildeten Strahlungsquelle (1) erzeugt wird.

19. Verfahren nach Anspruch 18, **dadurch gekennzeichnet, daß** als Laser (1) ein frequenzvervielfachter Festkörperlaser, ein Excimerlaser, ein anderer Gaslaser, ein frequenzvervielfachter Farbstofflaser oder ein frequenzvervielfachter lonenlaser verwendet wird.

20. Verfahren nach Anspruch 18, **dadurch gekennzeichnet, daß** als Blitzlampe eine ein Xenon-Gasgemisch oder ein Deuterlum-Gasgemisch enthaltende Blitzlampe verwendet wird.

21. Verfahren nach mindestens einem der vorangehenden Ansprüche, **dadurch gekennzeichnet, daß** als Bestrahlungsmuster (26) ein Muster aus parallelen Streifen, ein rechtwinkliges Gitter, ein Lochmuster, ein Muster aus mehreren konzentrischen Kreisllinien mit radial vom Zentrum ausgehenden und mit gleichem Winkelabstand angeordneten Linien oder ein aus zwei Linlenmustern bestehendes Moiré-Muster gewählt wird.

22. Verfahren nach Anspruch 21, **dadurch gekennzeichnet, daß** das Bestrahlungsmuster (26) zumindest teilweise durch die optische Abbildung einer Maske (4) mit Öffnungen in Form von parallelen Schlitzen oder regelmäßig angeordneten Löchern erzeugt wird.

23. Verfahren nach mindestens einem der vorangehenden Ansprüche, **dadurch gekennzeichnet, daß** das Bestrahlungsmuster (26) zumindest teilweise durch die optische Abbildung eines strukturierten Glases mit die Anregungsstrahlung (2) absorbierenden und/oder streuenden sowie für die Anregungsstrahlung (2) transparenten Bereichen erzeugt wird.

24. Verfahren nach mindestens einem der vorangehenden Ansprüche, **dadurch gekennzeichnet, daß** das Bestrahlungsmuster (26) zumindest teilweise durch Interferenz der Anregungsstrahlung (2) erzeugt wird, insbesondere durch Teilen und anschließendes Rekombinieren der von einer Strahlungsquelle (1) erzeugten Anregungsstrahlung (2) oder durch Bestrahlen des Gewebes (8a) mit einer Anregungsstrahlung (2), die von zwei aufeinander abgestimmte Strahlungsquellen (1) erzeugt wird.

25. Verfahren nach mindestens einem der vorangehenden Ansprüche, **dadurch gekennzeichnet, daß** das Bestrahlungsmuster (28) zumindest teilweise durch diffraktive optische Elemente, vorzugsweise Mikrollnsen, erzeugt wird.

26. Verfahren nach mindestens einem der vorangehenden Ansprüche, **dadurch gekennzeichnet, daß** das Bestrahlungsmuster (26) zumindest teilweise durch eine regelmäßige Anordnung von Mikrospiegeln erzeugt wird.

27. Verfahren nach mindestens einem der vorangehenden Ansprüche, **dadurch gekennzeichnet, daß** die Oberflächenform des Gewebes (8a) von einer Auswerteeinheit berechnet wird, die mittels der berechneten Oberflächenform einen Laser steuert.

28. Verfahren nach Anspruch 27, **dadurch gekennzeichnet, daß** der von der Auswerteeinheit gesteuerte Laser und die zur Fluoreszenzanregung des biologischen Gewebes (8a) verwendete Strahlungsquelle (1), vorzugsweise ein UV-Laser (1) übereinstimmen.

29. Vorrichtung zur Ermittlung der Oberflächenform von biologischem Gewebe, insbesondere zur Durchführung des Verfahrens nach einem der Ansprüche 1 bis 28, mit

- mindestens einer Strahlungsquelle (1) zum Erzeugen einer Anregungsstrahlung (2),
- Mitteln (4) zum Erzeugen eines Bestrahlungsmusters (26) aus der Anregungstrahlung (2) direkt auf dem Gewebe (8a), so daß die bestrahlten Gewebebereiche zur Emission eines aus Fluoreszenzstrahlung (14) bestehenden Fluoreszenzmusters (27) angeregt werden,
- mindestens einer Detektionsvorrichtung (12) zum Detektieren der vom Gewebe (8a) emittierten Fluoreszenzstrahlung (14), und
- einer Auswerteeinheit zur Berechnung der Oberflächenform des Gewebes (8a) aus der detektierten Fluoreszenzstrahlung (14).

**dadurch gekennzeichnet, daß** die Strahlungsquelle (1) eine Anregungsstrahlung (2) mit im wesentlichen im ultravioletten (UV) Wellenlängenbereich liegender Wellenlänge erzeugt.

30. Vorrichtung nach Anspruch 29, **dadurch gekennzeichnet, daß** die Strahlungsquelle (1) als Laser (1), vorzugsweise als frequenzvervielfachter Festkörperlaser, Excimerlaser, Gaslaser oder frequenzvervielfachter Farbstofflaser, oder als Blitzlampe, vorzugsweise mit einem Xenon- oder einem Deuterium-Gasgemisch gefüllt, ausgebildet ist.

31. Vorrichtung nach Anspruch 29 oder 30, **gekennzeichnet durch** mindestens eine weitere Strahlungsquelle (1) und/oder mindestens eine Einrichtung zur Aufteilung der Anregungsstrahlung (2), um das biologische Gewebe (8a) aus mindestens zwei Richtungen mit der Anregungsstrahlung (2) zu bestrahlen.

32. Vorrichtung nach mindestens einem der Ansprüche 29 bis 31, **gekennzeichnet durch** mindestens eine zusätzliche Detektionsvorrichtung (22) zum Detektieren der das Fluoreszenzmuster (27) bildenden Fluoreszenzstrahlung (14).

33. Vorrichtung nach mindestens einem der Ansprüche 29 bis 32, **gekennzeichnet durch** mindestens eine Lichtumlenkeinrichtung zur Umlenkung von Fluoreszenzstrahlung (14) zu einer Detektionsvorrichtung (12, 22).

34. Vorrichtung nach mindestens einem der Ansprüche 29 bis 33, **dadurch gekennzeichnet, daß** die Mittel (4) zum Erzeugen des Bestrahlungsmusters (26) eine Maske (4) mit Öffnungen in Form von parallelen Schlitzen oder regelmäßig angeordneten Löchern umfassen.

35. Vorrichtung nach mindestens einem der Ansprüche 29 bis 34, **dadurch gekennzeichnet, daß** die Mittel (4) zum Erzeugen des Bestrahlungsmusters (26) ein strukturiertes Glas mit die Anregungsstrahlung (2) absorbierenden und/oder streuenden sowie für die Anregungsstrahlung (2) transparenten Bereichen umfassen.

36. Vorrichtung nach mindestens einem der Ansprüche 29 bis 35, **dadurch gekennzeichnet, daß** die Mittel (4) zum Erzeugen des Bestrahlungsmusters (26) eine vorzugsweise regelmäßige Anordnung von quer zum Strahlengang der Anregungsstrahlung (2) angeordneten diffraktiven optischen Elementen, vorzugsweise Mikrolinsen, umfassen.

**37.** Vorrichtung nach mindestens einem der Ansprüche 29 bis 36, **dadurch gekennzeichnet, daß** die Mittel (4) zum Erzeugen des Bestrahlungsmusters (26) Mittel zum Erzeugen eines Interferenzmusters auf dem biologischen Gewebe (8a) umfassen.

**38.** Vorrichtung nach mindestens einem der Ansprüche 29 bis 37, **dadurch gekennzeichnet, daß** die Mittel (4) zum Erzeugen des Bestrahlungsmusters (26) mindestens ein Feld aus Mikrospiegeln umfassen.

**39.** Vorrichtung nach mindestens einem der Ansprüche 29 bis 38, **dadurch gekennzeichnet, daß** die Detektionsvorrichtung (12) eine CCD-Kamera (12) umfaßt.

**40.** Vorrichtung nach mindestens einem der Ansprüche 29 bis 39, **dadurch gekennzeichnet, daß** die Strahlungsquelle (1) hinsichtlich Intensität, Pulsdauer, Wiederholrate und Wellenlänge der Anregungsstrahlung (2) zur operativen Behandlung des biologischen Gewebes (8a), wie beispielsweise der bereichsweisen Abtragung einer Hornhaut (8a), ausgebildet ist.

**41.** Vorrichtung nach mindestens einem der Ansprüche 29 bis 40, **gekennzeichnet durch** einen Intensitätsabschwächer (15) oder einen Strahlaufweiter zwischen der mindestens einen Strahlungsquelle (1) und dem biologischen Gewebe (8a) zum Einführen und Herausnehmen aus dem Strahlengang der Anregungsstrahlung (2).

**42.** Vorrichtung nach mindestens einem der Ansprüche 29 bis 41, **gekennzeichnet durch** einen Eye-Tracker zum Ermitteln von Informationen über typische Augenbewegungen, mit deren Hilfe Anregungsstrahlung (2) dem Auge (8b) nachgeführt wird, um die Bestrahlungszeiten des Bestrahlungsmusters (26) auf die Hornhaut (8a) zu verlängern.

**43.** Vorrichtung nach mindestens einem der Ansprüche 29 bis 42, **gekennzeichnet durch** einen Eye-Tracker zur Bestimmung der Lage des Auges (8b) vor jeder Bestrahlung mit dem Bestrahlungsmuster (26) und/oder nach jeder Detektion des Fluoreszenzmusters (27).

**44.** Vorrichtung nach mindestens einem der Ansprüche 29 bis 43, **gekennzeichnet durch** einen Eye-Tracker zur Bestimmung der Lage des Auges (8b) während der Bestrahlung mit dem Bestrahlungsmuster (28) und/oder während der Datektion des Fluoreszenzmusters (27).

**45.** Vorrichtung nach einem der Ansprüche 29 bis 44, **dadurch gekennzeichnet, daß** ein Computer vorgesehen ist, der die Oberflächenform des Gewebes (8a) ermittelt, die zur Steuerung eines Lasers herangezogen wird.

**46.** Vorrichtung nach Anspruch 45, **dadurch gekennzeichnet, daß** der von dem Computer gesteuerte Laser und die zur Fluoreszenzanregung des biologischen Gewebes (8a) verwendete Strahlungsquelle (1), vorzugsweise ein UV-Laser (1), übereinstimmen.

**Claims**

**1.** A method for determining the surface form of biological tissue, **characterized in that** the tissue (8a) is directly irradiated with an irradiation pattern (26) produced with the aid of an excitation radiation (2) so that the irradiated tissue areas are excited to emit a fluorescent pattern (27) consisting of fluorescent radiation (14), which pattern is detected and evaluated in order to calculate the surface form of the tissue (8a).

**2.** The method according to claim 1, **characterized in that** the tissue to be measured (8a) is the cornea (8a) of an eye (8b) or the skin on a finger tip.

**3.** The method according to claim 2, **characterized in that** the tear film on the cornea (8a) is removed before the determination of the surface form of the cornea (8a).

**4.** The method according to claim 3, **characterized in that** prior to the determination of the surface form of the cornea (8a) the epithelial layer of the cornea (8a) is removed at least temporarily out of the beam path of the excitation radiation (2) in such a manner that the stromal tissue located under the epithelia] layer is directly irradiated with the irradiation pattern (26).

5. The method according to at least one of the preceding claims, **characterized in that** the tissue areas emitting the fluorescent radiation (14) are essentially excited with an excitation radiation (2) located in the ultraviolet (UV) wavelength range.

6. The method according to at least one of the preceding claims, **characterized in that** the biological tissue (8a) is irradiated from at least two directions with the excitation radiation (2).

7. The method according to at least one of the preceding claims, **characterized in that** the fluorescent radiation (14) is detected with at least one detection device (12, 22), preferably a CCD camera (12, 22).

8. The method according to claim 7, **characterized in that** the fluorescent radiation is detected in a locally resolved manner.

9. The method according to at least one of the preceding claims, **characterized in that** the fluorescent radiation (14) is detected under an angle ($\alpha$) different from the direction of irradiation.

10. The method according to at least one of the preceding claims, **characterized in that** the fluorescent radiation (14) is diverted at least partially by a light diverting device to a detection device (12, 22) where it is detected.

11. The method according to at least one of the preceding claims, **characterized in that** the excitation radiation (2) is selected to be in the wavelength range of 150 nm to 370 nm.

12. The method according to at least one of the preceding claims, **characterized in that** the emission time of the excitation radiation (2) is selected to be between 1 fs and several seconds,

13. The method according to at least one of claims 2 to 12, **characterized in that** an eye-tracker records information about typical courses of movement of the eye, with the aid of which information the excitation radiation (2) is readjusted with respect to the eye (8b) in order to realise long irradiation times.

14. The method according to at least one of the preceding claims 2 to 12, **characterized in that** a determination of the position of the eye (8b) is carried out with an eye-tracker before each irradiation with the irradiation pattern (26) and after each detection of the fluorescent pattern (27)

15. The method according to at least one of claims 2 to 12, **characterized in that** the position of the eye (8b) during the irradiation with the irradiation pattern (26) and during the detection of the fluorescent pattern (27) is determined by an eye-tracker and that the irradiation or the detection is halted upon a change of position during the irradiation with the irradiation pattern (26) or during the detection of the fluorescent pattern (27) and a new irradiation of the cornea (8a) of the eye (8b) is carried out with subsequent detection of the fluorescent pattern (27).

16. The method according to at least one of the preceding claims, **characterized in that** the irradiation pattern (26) is projected at a repetition rate between 1 Hz and 1 MHz onto the tissue (8a) for each measurement, that is composed of an irradiation with subsequent detection of the fluorescent pattern (27).

17. The method according to at least one of the preceding claims, **characterized in that** the energy of the excitation radiation (2) is selected between 1 µJ and 1 J.

18. The method according to at least one of the preceding claims, **characterized in that** the excitation radiation (2) is generated by a radiation source (1) designed as a laser (1) or a flash lamp.

19. The method according to claim 18, **characterized in that** a frequency-multiplied solid laser, an excimer laser, another gas laser, a frequency-multiplied dye laser or a frequency-multiplied ion laser is used as laser.

20. The method according to claim 18, **characterized in that** a flash lamp containing a gaseous xenon mixture or a gaseous deuterium mixture is used as flash lamp.

21. The method according to at least one of the preceding claims, **characterized in that** a pattern of parallel strips, a rectangular grid, a perforated pattern, a pattern consisting of several concentric circular lines with lines emanating radially from the centre and arranged with the same angular interval or a moire pattern consisting of two line patterns is selected as irradiation pattern (26).

22. The method according to claim 21, **characterized in that** the irradiation pattern (26) is produced at least partially by the optical imaging of a mask (4) with perforations in the form of parallel slits or regularly arranged perforations.

23. The method according to at least one of the preceding claims, **characterized in that** the irradiation pattern (26) is generated at least partially by the optical imaging of a structured glass with areas that absorb and/or scatter the excitation radiation (2) and are transparent for the excitation radiation (2).

24. The method according to at least one of the preceding claims, **characterized in that** the irradiation pattern (26) is generated at least partially by interference of the excitation radiation (2), in particular by means of a dividing and subsequent recombining of the excitation radiation (2) generated by a radiation source (1) or by means of irradiating the tissue (8a) with an excitation radiation (2) produced by two radiation sources (1) that are coordinated with each other.

25. The method according to at least one of the preceding claims, **characterized in that** the irradiation pattern (26) is generated at least partially by diffractive optical elements, preferably microlenses.

26. The method according to at least one of the preceding claims, **characterized in that** the irradiation pattern (26) is generated at least partially by a regular arrangement of micromirrors.

27. The method according to at least one of the preceding claims, **characterized in that** the surface form of the tissue (8a) is calculated by an evaluating unit that controls a laser by means of the calculated surface form.

28. The method according to claim 27, **characterized in that** the laser controlled by the evaluating unit and the radiation source (1) used for the fluorescence excitation of the biological tissue (8a), preferably a UV laser (1), are identical.

29. A device for determining the surface form of biological tissue, in particular for carrying out the method according to one of claims 1 to 28, with

   - At least one radiation source (1) for generating an excitation radiation (2),

   - Means (4) for producing an irradiation pattern (26) from the excitation radiation (2) directly on the tissue (8a) so that the irradiated tissue areas are excited to emit a fluorescent pattern (27) consisting of fluorescent radiation (14),

   - At least one detection device (12) for detecting the fluorescent radiation (14) emitted by the tissue (8a), and

   - An evaluating unit for calculating the surface form of the tissue. (8a) from the detected fluorescent radiation (14), **characterized in that** the radiation source (1) generates an excitation radiation (2) with a wavelength located substantially in the ultraviolet (UV) wavelength range.

30. The device according to claim 29, **characterized in that** the radiation source (1) is designed as a laser (1), preferably as a frequency-multiplied solid laser, excimer laser, gas laser or frequency-multiplied dye laser or as a flash lamp, preferably filled with a gaseous xenon mixture or gaseous deuterium mixture.

31. The device according to claim 29 or 30, **characterized by** at least one further radiation source (1) and/or at least one device for splitting the excitation radiation (2) in order to irradiate the biological tissue (8a) from at least two directions with the excitation radiation (2).

32. The device according to at least one of claims 29 to 31, **characterized by** at least one additional detection device (22) for detecting the fluorescent radiation (14) forming the fluorescent pattern (27)

33. The device according to at least one of claims 29 to 32, **characterized by** at least one light deflection device for deflecting fluorescent radiation (14) to a detection device (12, 22).

**EP 1 105 038 B1**

34. The device according to at least one of claims 29 to 33, **characterized in that** the means (4) for producing the irradiation pattern (26) comprises a mask (4) with openings in the form of parallel slits or regularly arranged perforations.

35. The device according to at least one of claims 29 to 34, **characterized in that** the means (4) for producing the irradiation pattern (26) comprises a structured glass with areas that absorb and/or scatter the excitation radiation (2) and are transparent for the excitation radiation (2).

36. The device according to at least one of claims 29 to 35, **characterized in that** the means (4) for producing the irradiation pattern (26) comprises a preferably regular arrangement of diffractive, optical elements, preferably microlenses, arranged transversely to the beam path of the excitation radiation (2).

37. The device according to at least one of claims 29 to 36, **characterized in that** the means (4) for producing the irradiation pattern (26) comprises means for producing an interference pattern on the biological tissue (8a).

38. The device according to at least one of claims 29 to 37, **characterized in that** the means (4) for producing the irradiation pattern (26) comprises at least one field of micromirrors.

39. The device according to at least one of claims 29 to 38, **characterized in that** the detection device (12) comprises a CCD camera (12).

40. The device according to at least one of claims 29 to 39, **characterized in that** the radiation source (1) is designed as regards intensity, pulse duration, repetition rate and wavelength of the excitation radiation (2) for the operative treatment of the biological tissue (8a), such as, e.g., the areawise removal of a cornea (8a).

41. The device according to at least one of claims 29 to 40, **characterized by** an intensity attenuator (15) or a beam widener between the at least one radiation source (1) and the biological tissue (8a) which intensity attenuator or beam widener is to be introduced into and removed from the beam path of the excitation radiation (2).

42. The device according to at least one of claims 29 to 41, **characterized by** an eye-tracker for determining information about typical eye movements with the aid of which excitation radiation (2) is readjusted with respect to the eye (8b) in order to lengthen the irradiation times of the irradiation pattern (26) on the cornea (8a).

43. The device according to at least one of claims 29 to 42, **characterized by** an eye-tracker for determining the position of the eye (8b) before each irradiation with the irradiation pattern (26) and/or after each detection of the fluorescent pattern (27).

44. The device according to at least one of claims 29 to 43, **characterized by** an eye-tracker for determining the position of the eye (8b) during the irradiation with the irradiation pattern (26) and/or during the detection of the fluorescent pattern (27).

45. The device according to one of claims 29 to 44, **characterized in that** a computer is provided which determines the surface form of the tissue (8a) which form is used to control a laser.

46. The device according to claim 45, **characterized in that** the laser controlled by the computer and the radiation source, preferably an UV laser (1), used for the fluorescent excitation of the biological tissue (8a) are identical.

**Revendications**

1. Procédé de détermination de la forme de surface de tissus biologiques, **caractérisé en ce qu'**il comporte les étapes qui consistent à faire rayonner directement sur le tissu (8a) un modèle (26) de rayonnement produit à l'aide d'un rayonnement (2) d'excitation, de sorte que les zones de tissu exposées au rayonnement soient excitées en vue d'une émission d'un modèle (27) fluorescent constitué de rayonnement (14) fluorescent, modèle qui est alors détecté et exploité pour le calcul de la forme de surface du tissu (8a).

2. Procédé suivant la revendication 1, **caractérisé en ce que** le tissu (8a) à mesurer est la peau (8a) de cornée d'un oeil (8b) ou la peau de pulpe du doigt.

3. Procédé suivant la revendication 2, **caractérisé en ce que**, avant la détermination de la forme de surface de la peau (8a) de cornée, on enlève la pellicule de larme de la peau (8a) de cornée.

4. Procédé suivant la revendication 3, **caractérisé en ce que**, avant la détermination de la forme de surface de la peau (8a) de cornée, la couche épitéliale de la peau (8a) de cornée est enlevée au moins temporairement de la marche des rayons du rayonnement (2) d'excitation, de sorte que le tissu stroma se trouvant sous la couche épitéliale est directement soumis au rayonnement du modèle (26) de rayonnement.

5. Procédé suivant l'une au moins des revendications précédentes, **caractérisé en ce que** la zone de tissu émettant le rayonnement fluorescent est excitée par principalement un rayonnement (2) d'excitation se trouvant dans la région des longueurs d'onde de l'ultraviolet (UV).

6. Procédé suivant au moins l'une des revendications précédentes, **caractérisé en ce que** l'on fait rayonner le rayonnement (2) d'excitation sur le tissu (8a) biologique à partir d'au moins deux directions.

7. Procédé suivant au moins l'une des revendications précédentes, **caractérisé en ce que** le rayonnement (14) fluorescent est détecté par au moins un dispositif (12, 22) de détection, de préférence une caméra (12, 22) CCD.

8. Procédé suivant la revendication 7, **caractérisé en ce que** le rayonnement (14) fluorescent est détecté avec une résolution spatiale.

9. Procédé suivant au moins l'une des revendications précédentes, **caractérisé en ce que** le rayonnement (14) fluorescent est détecté suivant un angle ($\alpha$) différent de la direction de rayonnement.

10. Procédé suivant au moins l'une des revendications précédentes, **caractérisé en ce que** le rayonnement (14) fluorescent est dévié au moins en partie par un dispositif de déviation de lumière vers un dispositif (12, 22) de détection pour y être détecté.

11. Procédé suivant au moins l'une des revendications précédentes, **caractérisé en ce que** le rayonnement (2) d'excitation est sélectionné dans la région des longueurs d'onde comprise entre 150 nm et 370 nm.

12. Procédé suivant au moins l'une des revendications précédentes, **caractérisé en ce que** la durée d'émission du rayonnement (2) d'excitation est choisie entre une femto seconde et plusieurs secondes.

13. Procédé suivant au moins l'une des revendications 2 à 12, **caractérisé en ce qu'**un Eye-Tracker dessine des informations concernant des allures de déplacement classiques de l'oeil, à l'aide desquelles le rayonnement (2) d'excitation est guidé vers l'oeil (8b), afin d'obtenir des temps de rayonnement longs.

14. Procédé suivant au moins l'une des revendications 2 à 12, **caractérisé en ce qu'**une détermination de position de l'oeil (8b) est effectuée à l'aide d'un Eye-Tracker avant chaque rayonnement avec le modèle (26) de rayonnement et après chaque détection du modèle (27) de fluorescence.

15. Procédé suivant au moins l'une des revendications 2 à 12, **caractérisé en ce que** la position de l'oeil (8b), pendant le rayonnement avec le modèle (26) de rayonnement et pendant la détection du modèle (27) de fluorescence est déterminée au moyen d'un Eye-Tracker et **en ce que** lors d'une modification de la position, pendant le rayonnement avec le modèle (26) de rayonnement ou pendant la détection du modèle (27) de fluorescence, le rayonnement ou la détection est arrêtée et un nouveau rayonnement de la peau (8a) de cornée de l'oeil (8b) avec une détection par la suite du modèle (27) de fluorescence est mis en oeuvre.

16. Procédé suivant au moins l'une des revendications précédentes, **caractérisé en ce que** le modèle (26) de rayonnement pour chaque mesure, qui se compose d'un rayonnement avec une détection qui suit du modèle (27) de fluorescence, est projeté sur le tissu (8a) à une fréquence de répétition comprise entre 1 Hz et 1 MHz.

17. Procédé suivant au moins l'une des revendications précédentes, **caractérisé en ce que** l'énergie du rayonnement (2) d'excitation est sélectionné entre 1 $\mu$J et 1 J.

18. Procédé suivant au moins l'une des revendications précédentes, **caractérisé en ce que** le rayonnement (2) d'excitation est produit par une source (1) de rayonnement formée sous la forme d'un laser (1) ou d'une lampe à décharge.

**19.** Procédé suivant la revendication 18, **caractérisé en ce que** l'on utilise comme laser (1) un laser à corps fixe générateur d'harmonique, un laser excimère, un autre laser à gaz, un laser couleur générateur. d'harmonique ou un laser ionique générateur d'harmonique.

**20.** Procédé suivant la revendication 18, **caractérisé en ce que** l'on utilise comme lampe à décharge une lampe à décharge contenant un mélange gazeux au Xénon ou un mélange gazeux au deutérium.

**21.** Procédé suivant au moins l'une des revendications précédentes, **caractérisé en ce que** l'on sélectionne comme modèle (26) de rayonnement un modèle constitué de bandes parallèles, une grille à angle droit, un modèle de trous, un modèle constitué de plusieurs lignes circulaires concentriques et de lignes partant radialement à partir du centre et disposées à des distances angulaires égales les unes des autres ou d'un modèle de Moiré constitué de deux modèles de lignes.

**22.** Procédé suivant la revendication 21, **caractérisé en ce que** le modèle (26) de rayonnement est produit au moins en partie par la formation optique d'un masque (4) comportant des ouvertures sous la forme de fentes parallèles ou de trous disposés de manière régulière.

**23.** Procédé suivant au moins l'une des revendications précédentes, **caractérisé en ce que** le modèle (26) de rayonnement est produit au moins en partie par la formation optique d'un verre structuré comportant des régions absorbant le rayonnement (2) d'excitation et/ou dispersant le rayonnement (2) d'excitation ainsi que de région transparentes pour le rayonnement (2) d'excitation.

**24.** Procédé suivant au moins l'une des revendications précédentes, **caractérisé en ce que** le modèle (26) de rayonnement est produit au moins en partie par interférence du rayonnement (2) d'excitation, notamment par partition et recombinaison ensuite du rayonnement (2) d'excitation produit à partir d'une source (1) de rayonnement ou par rayonnement sur le tissu (8a) avec un rayonnement (2) d'excitation, qui est produit à partir de deux sources (1) de rayonnement mutuellement accordées.

**25.** Procédé suivant au moins l'une des revendications précédentes, **caractérisé en ce que** le modèle (26) de rayonnement est produit au moins en partie par des éléments optiques de diffraction, de préférence des micro-lentilles.

**26.** Procédé suivant au moins l'une des revendications précédentes, **caractérisé en ce que** le modèle (26) de rayonnement est produit au moins en partie par une disposition régulière de micro-miroirs.

**27.** Procédé suivant au moins l'une des revendications précédentes, **caractérisé en ce que** la forme de surface du tissu (8a) est calculée à partir d'une unité d'exploitation, qui commande un laser au moyen de la forme de surface calculée.

**28.** Procédé suivant la revendication 27, **caractérisé en ce que** le laser commandé par l'unité d'exploitation et la source (1) de rayonnement, de préférence un laser (1) UV, utilisée pour l'excitation fluorescente du tissu (8a) biologique sont accordés mutuellement.

**29.** Dispositif pour déterminer la forme de surface de tissu biologique, notamment pour la mise en oeuvre du procédé suivant l'une des revendications 1 à 28, comportant

- au moins une source (1) de rayonnement destinée à produire un rayonnement (2) d'excitation,
- des moyens (4) destinés à produire un modèle (26) de rayonnement à partir du rayonnement (2) d'excitation directement sur le tissu (8a), de sorte que la région de tissu qui reçoit le rayonnement est excitée en vue d'une émission d'un modèle (27) fluorescent constitué de rayonnement (14) fluorescent,
- au moins un dispositif (12) de détection destiné à détecter le rayonnement (14) fluorescent émis du tissu (8a), et
- une unité d'exploitation destinée à calculer la forme de surface du tissu (8a) à partir du rayonnement (14) fluorescent détecté,

**caractérisé en ce que** la source (1) de rayonnement produit un rayonnement (2) d'excitation ayant des longueurs d'onde se trouvant sensiblement dans le domaine des longueurs d'onde de l'ultraviolet (UV).

**30.** Dispositif suivant la revendication 29, **caractérisé en ce que** la source (1) de rayonnement est sous la forme d'un laser (1), de préférence un laser à semi-conducteur générateur d'harmonique, un laser excimère, un laser à gaz ou un laser couleur générateur d'harmonique, ou sous la forme d'une lampe à éclair, de préférence une lampe à éclair emplie d'un mélange gazeux à base de xénon ou de deutérium.

**31.** Dispositif suivant la revendication 29 ou 30, **caractérisée par** au moins une source (1) de rayonnement supplémentaire et/ou au moins un dispositif pour diviser le rayonnement (2) d'excitation, afin d'envoyer sur le tissu (8a) biologique un rayonnement (2) d'excitation suivant au moins deux directions.

**32.** Dispositif suivant au moins l'une des revendications 29 à 31, **caractérisé par** au moins un dispositif (22) de détection supplémentaire en vue de détecter le rayonnement (14) fluorescent formant le modèle (27) fluorescent.

**33.** Dispositif suivant au moins l'une des revendications 29 à 32, **caractérisé par** au moins un dispositif de déviation de lumière en vue de dévier le rayonnement (14) fluorescent vers un dispositif (12, 22) de détection.

**34.** Dispositif suivant au moins l'une des revendications 29 à 33, **caractérisé en ce que** les moyens (4) destinés à produire le modèle (28) de rayonnement comporte un masque (4) ayant des ouvertures sous la forme de fentes parallèles ou de trous disposés de manière régulière.

**35.** Dispositif suivant au moins l'une des revendications 29 à 34, **caractérisé en ce que** les moyens (4) destinés à produire le modèle (26) de rayonnement comporte un verre structuré ayant des régions absorbant le rayonnement (2) d'excitation et/ou dispersant le rayonnement (2) d'excitation ainsi que des régions transparentes pour le rayonnement (2) d'excitation.

**36.** Dispositif suivant au moins l'une des revendications 29 à 35, **caractérisé en ce que** les moyens (4) destinés à produire le modèle (26) de rayonnement comporte un agencement de préférence régulier d'éléments optiques diffractifs, de préférence des micro-lentilles, disposés transversalement au rayon du rayonnement (2) d'excitation.

**37.** Dispositif suivant au moins l'une des revendications 29 à 36, **caractérisé en ce que** les moyens (4) destinés à produire le modèle (26) de rayonnement comportent des moyens destinés à produire un modèle d'interférence sur le tissu (8a) biologique.

**38.** Dispositif suivant au moins l'une des revendications 29 à 37, **caractérisé en ce que** les moyens (4) destinés à produire le modèle (26) de rayonnement comportent au moins un champ de micro-miroirs.

**39.** Dispositif suivant au moins l'une des revendications 29 à 38, **caractérisé en ce que** le dispositif (12) de détection comporte une caméra (12) CCD.

**40.** Dispositif suivant au moins l'une des revendications 29 à 39, **caractérisé en ce que** la source (1) de rayonnement, en ce qui concerne l'intensité, la durée d'impulsion, la fréquence de répétition et la longueur d'onde du rayonnement (2) d'excitation, est constituée pour un traitement fonctionnel du tissu (8a) biologique, comme par exemple un enlèvement, par portion, d'une peau (8a) de cornée.

**41.** Dispositif suivant au moins l'une des revendications 29 à 40, **caractérisé par** un dispositif (15) de diminution d'intensité ou un amplificateur de rayonnement disposé entre ladite au moins une source de rayonnement et le tissu (8a) biologique en vue d'introduire et d'extraire du trajet de rayonnement du rayonnement (2) d'excitation.

**42.** Dispositif suivant au moins l'une des revendications 29 à 41, **caractérisé par** un Eye-Tracker pour déterminer des informations concernant des déplacements typiques d'oeil, à l'aide desquelles le rayonnement (2) d'excitation est envoyé vers l'oeil (8b), afin de prolonger la durée de rayonnement du modèle (26) de rayonnement sur la peau (8a) de cornée.

**43.** Dispositif suivant au moins l'une des revendications 29 à 42, **caractérisé par** un Eye-Tracker destiné à déterminer la position de l'oeil (8b) avant chaque rayonnement avec le modèle (26) de rayonnement et/ou après chaque détection du modèle (27) de fluorescence.

**44.** Dispositif suivant au moins l'une des revendications 29 à 43, **caractérisé par** un Eye-Tracker destiné à déterminer la position de l'oeil (8b) pendant le rayonnement avec le modèle (26) de rayonnement et/ou pendant la détection du modèle (27) de fluorescence.

**45.** Dispositif suivant au moins l'une des revendications 29 à 44, **caractérisé en ce qu'**il est prévu un ordinateur, qui détermine la forme de surface du tissu (8a), qui est utilisée pour commander un laser.

**46.** Dispositif suivant la revendication 45, **caractérisé en ce que** le laser commandé par ordinateur et la source (1) de courant utilisée pour l'excitation fluorescente du tissu (8a) biologique, de préférence un laser (1) UV, sont accordés mutuellement.

Fig. 1

EP 1 105 038 B1

EP 1 105 038 B1

Fig. 2

Fig. 3

Fig. 4